(19) Europäisches Patentamt
European Patent Office
Office européen des brevets

(11) Publication number: **0 062 873**
**A1**

(12) **EUROPEAN PATENT APPLICATION**

(21) Application number: 82102893.3

(22) Date of filing: 05.04.82

(51) Int. Cl.³: C 07 D 471/04
A 61 K 31/44
//C07D211/52, C07D211/70,
(C07D471/04, 249/00, 221/00)

(30) Priority: 13.04.81 US 253137

(43) Date of publication of application:
20.10.82 Bulletin 82/42

(84) Designated Contracting States:
AT BE CH DE FR GB IT LI NL SE

(71) Applicant: SCHERING CORPORATION
2000 Galloping Hill Road
Kenilworth, New Jersey 07033(US)

(72) Inventor: Gold, Elijah H.
10 Roosevelt Avenue
West Orange New Jersey 07052(US)

(72) Inventor: Berger, Joel G.
54 Park Avenue
Verona New Jersey 07044(US)

(72) Inventor: Chang, Wei
63 West Cedar Street
Livingston New Jersey 07039(US)

(74) Representative: Antony, Fritz, Dr. et al,
P.O. Box 601 Winkelriedstrasse 35
CH-6002 Lucerne(CH)

(54) **1-Substituted-4-phenyl-polyhydro-pyridines and -pyrrolidines, processes for their preparation and pharmaceutical compositions containing them.**

(57) Compounds of the formula

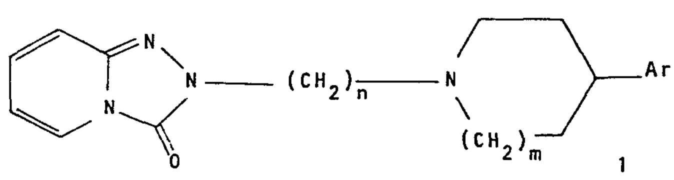

1

wherein Ar is a phenyl group or a phenyl group substituted by one or more members of the group consisting of halogen atoms or lower alkyl, halolower alkyl, hydroxy or lower alkoxy groups, "lower" indicating groups having from 1 to 5 carbon atoms;
*m* is zero or 1, *n* is 2 or 3;
and their dehydro derivatives having a double bond in the azacyclic ring extending from the carbon atom having the Ar substituent;
and the pharmaceutically acceptable acid addition salts thereof;
have analgesic properties. Processes for their preparation and pharmaceutical compositions containing them are also claimed.

EP 0 062 873 A1

Croydon Printing Company Ltd.

# 1-SUBSTITUTED-4-PHENYL-POLYHYDRO-PYRIDINES AND -PYRROLIDINES, PROCESSES FOR THEIR PREPARATION AND PHARMACEUTICAL COMPOSITIONS CONTAINING THEM.

The invention relates to 1-substituted-4-phenyl-polyhydro-pyridines and -pyrrolidines, to their pharmaceutically acceptable acid addition salts, to methods for their preparation and to pharmaceutical compositions containing them.

According to the invention we provide compounds of the following formula:

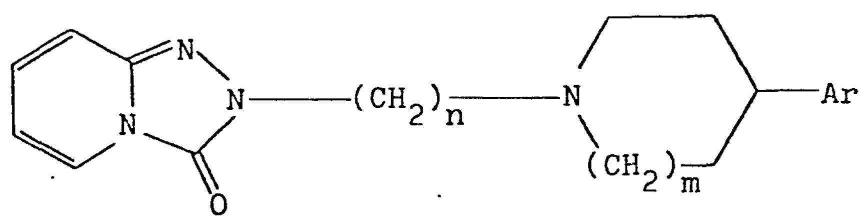

I

wherein Ar is a phenyl group or a phenyl group substituted by one or more members of the group consisting of halogen atoms or lower alkyl, halolower alkyl, hydroxy or lower alkoxy groups, "lower" indicating groups having from 1 to 5 carbon atoms;

m is zero or 1, n is 2 or 3;

and their dehydro derivatives having a double bond in the azacyclic ring extending from the carbon atom having the Ar substituent;

and the pharmaceutically acceptable acid addition salts thereof.

The azacyclic ring referred to in the preceding paragraph is of course the ring of the formula

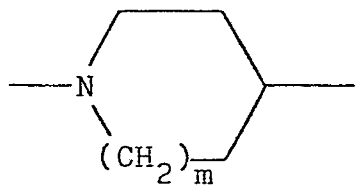

wherein m is zero or 1.

Two preferred sub-groups of compounds of formula I have the formulae:

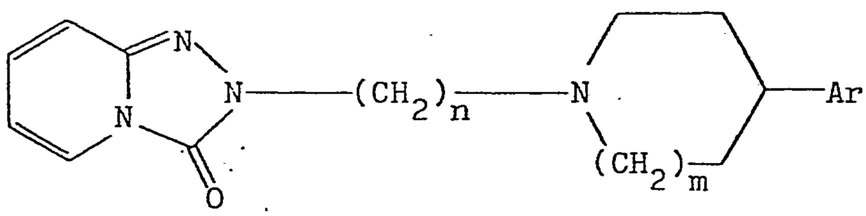

IA

and

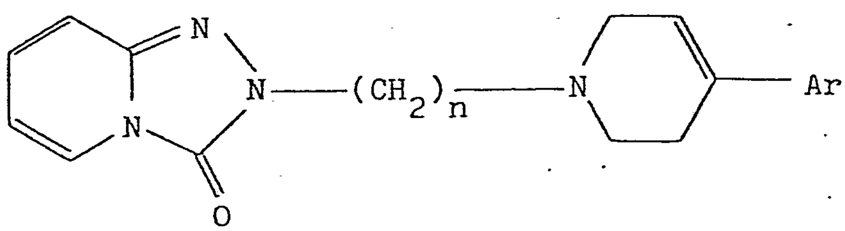

II

wherein Ar, m and n are as defined above.

Particularly preferred compounds of the formula I, on account of their very favourable pharmacological properties, include 2-[3-(4-phenylpiperidino)-propyl]-1,2,4-triazolo[4,3-a]pyridin-3-(2H)-one, m.p. 102-104$^{o}$C., and 2-{ 3-[1-(1,2,5,6-tetrahydro-4-o-methylphenyl-pyridyl)]-propyl} -1,2,4-triazolo[4,3-a]pyridin-3-(2H)-one, m.p. 106-107$^{o}$C.

Other preferred compounds of formula I include:

2-{ 3-[1-(1,2,5,6-Tetrahydro-4-p-chlorophenyl-pyridyl)]--propyl} -1,2,4-triazolo[4,3-a]pyridin-3-(2H)-one, m.p. 123-124°C.,

2-{ 3-[1-(1,2,5,6-tetrahydro-4-*p*-methylphenyl-pyridyl)]-propyl} -1,2,4-triazolo[4,3-a]pyridin-3-(2*H*)-one, m.p. 102-104°C.,

2-[3-(4-*p*-chlorophenyl-piperidino)-propyl]-1,2,4-triazolo[4,3-a]pyridin-3-(2*H*)-one, m.p. 79.5-82°C.,

2-[3-(4-*m*-chlorophenyl-piperidino)-propyl]-1,2,4-triazolo-[4,3-a]pyridin-3-(2*H*)-one, m.p. 134-136°C.,

2-[3-(4-*p*-methylphenyl-piperidino)-propyl] -1,2,4-triazolo[4,3-a] pyridin-3-(2*H*)-one, m.p. 106-107.5°C.,

2-[3-(4-*m*-methylphenyl-piperidino)-propyl] -1,2,4-triazolo[4,3-a]pyridin-3-(2*H*)-one, m.p. 106-108°C.,

2-{ 3-[1-(1,2,5,6-tetrahydro-4-*m*-chlorophenylpyridyl)]-propyl} -1,2,4-triazolo[4,3-a]pyridin-3-(2*H*)-one, m.p. 94-96°C.,

2- [3-(4-*p*-fluorophenyl-piperidino)-propyl]-1,2,4-triazolo[4,3-a]pyridin-3-[2*H*]-one, m.p. 89-91°C.,

2-[3-(4-*p*-methoxyphenyl-piperidino)-propyl]-1,2,4-triazolo [4,3-a]pyridin-3-(2*H*)-one, m.p. 99-101°C.,

2-{ 3-[1-(3-phenylpyrrolidinyl)]-propyl} -1,2,4-triazolo[4,3-a] pyridin-3-(2*H*)-one, m.p. 109-111°C.,

2-{ 3-[1-(1,2,5,6-tetrahydro-3-*o*-trifluoromethylphenyl-pyridyl)]-propyl} -1,2,4-triazolo[4,3-a]pyridin-3-(2*H*)-one, m.p. 124.5-126°C., and

2-[3-(4-*m*-hydroxyphenyl-piperidino)-propyl]-1,2,4-triazolo[4,3-a] pyridin-3-(2*H*)-one, m.p. 137-138°C.

Preferred pharmaceutically acceptable acid addition salts include those formed with hydrochloric, hydrobromic, nitric, sulfuric, maleic, fumaric, tartaric, citric or succinic acid.

According to a further feature of the invention, we provide a process for the preparation of a compound of the formula I or pharmaceutically acceptable acid addition salt thereof, which comprises either

a) reacting a compound of the formula

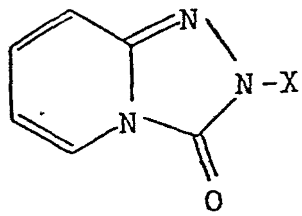

III

with a compound of the formula

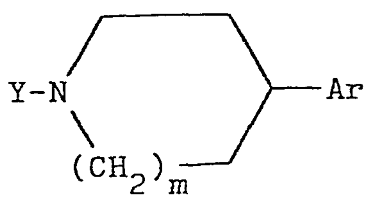

IV

or with a dehydro derivative thereof having a double bond in the azacyclic ring extending from the carbon atom having the Ar substituent;

wherein one of X and Y is a group of the formula

$$-(CH_2)_n-Z$$

where Z is a reactive ester group, and the other of X and Y is an atom or group capable of being eliminated with Z;

and m, n and Ar are as defined above;

or

b) — for the preparation of a dehydro derivative of a compound of the formula I having a double bond in the azacyclic ring extending from the carbon atom having the Ar substituent — eliminating a compound of the formula WH from a compound of the formula

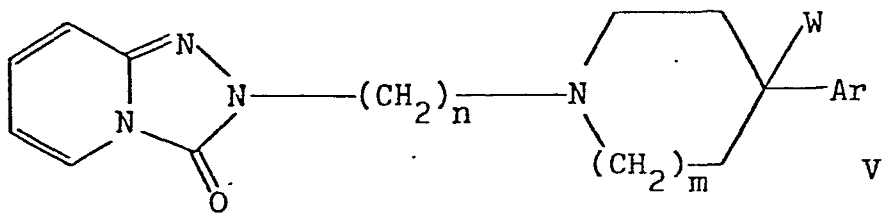

V

wherein $m$, $n$ and Ar are as defined above and W is a hydroxy group or an esterified hydroxy group;
whereafter the product of the formula I is isolated as the free base or as a pharmaceutically acceptable acid addition salt thereof.

In one particularly preferred embodiment of process a), X is a hydrogen atom and Y is the group $-(CH_2)_n-Z$, where Z is a halogen atom (such as bromine or especially chlorine) and $n$ is as defined above. Under these circumstances, the reaction is preferably carried out in the presence of a strong base such as an alkali metal amide or preferably hydride, especially sodium hydride, and in the presence of an anhydrous organic solvent, preferably a polar solvent such as a dialkylamide, e.g. dimethylacetamide or preferably dimethylformamide. The reaction is preferably carried out at elevated temperature, e.g. 80-120°C.

In a particularly preferred embodiment of this process, the compound of the formula III is dissolved in the solvent and the solution is heated to about 90°C. A dispersion of sodium hydride is then added in small quantities and, after the sodium derivative of the compound of the formula III has

been formed, the compound of the formula IV is added. The reaction mixture is heated until reaction is substantially complete, whereafter the product is isolated in the normal manner.

In another particularly preferred embodiment of process a), X is the group of the formula $-(CH_2)_n-Z$, where Z is preferably a halogen atom (such as bromine or especially chlorine) and n is as defined above, and Y is a hydrogen atom or a group that can be displaced in the reaction, for example a benzyl group. The reaction is preferably effected in an inert organic solvent, preferably in the presence of an acid-binding agent and if desired in the presence of a promoter. The organic solvent may be polar or moderately polar, for example dimethylformamide or dimethylacetamide. The acid binding agent may be an inorganic base, for example anhydrous potassium carbonate, but is preferably an organic base, preferably a tertiary amine such as pyridine or triethylamine. The promoter may be a halide salt in which the halogen atom has a higher molecular weight than the halogen Z, for example an alkali metal or alkaline earth metal iodide, in particular potassium iodide. The reaction is preferably carried out at elevated temperature, e.g. at about 80 to 120°C., until the reaction is substantially complete. The product can then be isolated in the usual manner.

Compounds of formula IV wherein Y is a hydrogen atom or a benzyl group can be prepared for example from a 1-benzyl-4-piperidone by reaction with an aryl Grignard reagent ArMgHal, wherein Ar is as defined above and Hal is a halogen atom, preferably bromine, to give the corresponding 1-benzyl-4-hydroxy-4-Ar-piperidine. This compound can be simply dehydrated with strong acid to a compound of the formula

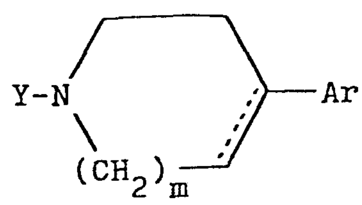

IVA

wherein Y is a benzyl group, m is 1, Ar is as defined above and the dotted line indicates a double bond; this compound can be reduced by hydrogen and a catalyst to a 4-Ar-piperidine of the formula IVA in which Y is a hydrogen atom, m is 1, Ar is as defined above and the dotted line indicates a single bond. Alternatively, the 1-benzyl-4-hydroxy--4-Ar-piperidine can be de-benzylated by treatment with hydrogen and a catalyst and the resulting 4-hydroxy-4-Ar-piperidine can be treated with strong acid to give a compound of the formula IVA wherein Y is a hydrogen atom, m is 1, Ar is as defined above and the dotted line indicates a double bond. The dehydration can be effected for example by means of hydrochloric or sulfuric acid in an alcohol as solvent.

Compounds of the formula IVA wherein Y is a hydrogen atom, m is 1, Ar is as defined above and the dotted line indicates a double bond can be prepared also by reacting an α-methylstyrene in a two-stage reaction, firstly with formaldehyde and ammonium chloride and secondly with a strong mineral acid such as hydrochloric acid, as described in Belgian Patent 577,977.

Compounds of the formula IVA wherein Y is a group of the formula $-(CH_2)_n-Z$, in particular those wherein Z is a chlorine atom, m is 1, Ar and n are as defined above and the dotted line is a single or double bond, can be prepared as described

by Pelz and Proteva, Coll.Czech.Chem.Comm., 32, 2840 (1967).

Compounds of the formula IV wherein m is zero can be prepared by known methods analogous to methods mentioned above.

Compounds of the formula III wherein X is a group $-(CH_2)_n$-Hal, wherein n is as defined above and Hal is a halogen, especially chlorine, can be prepared as described in USP 3,381,009.

In one particularly preferred embodiment of process b), W is a hydroxy group and the elimination of the compound of the formula WH is effected at moderate temperature under mildly acidic conditions provided for example by a dilute mineral acid. Thus the compound of the formula V can be dissolved in an inert water-miscible organic solvent (e.g. a lower alkanol such as ethanol or methanol) and the mineral acid as an aqueous solution (for example hydrochloric or sulfuric) can be added thereto and the mixture left to stand at 25-50°C. until the dehydration is substantially complete. The product of the formula I having a double bond in the azacyclic ring extending from the carbon atom having the Ar substituent can then be isolated in the normal manner either as an acid addition salt or as the free base.

Compounds of the formula V can be prepared for example by reaction of a corresponding piperidone with an aryl Grignard reagent ArMgHal, wherein Ar is as defined above and Hal is a halogen atom, preferably bromine, substantially as described above for the preparation of an intermediary 1-benzyl-4-hydroxy-4-Ar-piperidine.

The following examples illustrate this invention:

EXAMPLE 1

2-{3-[1-(1,2,5,6-Tetrahydro-4-o-methylphenyl-pyridyl)]propyl}-1,2,4-triazolo[4,3-a]pyridin-3(2H)-one.

A) 1-Benzyl-4-hydroxy-4-(o-methylphenyl)piperidine hydrochloride.

To a Grignard reagent prepared from 5.3 g. of magnesium turnings and 37 g. of 1-bromo-2-methyl benzene in 150 ml. of dry tetrahydrofuran add dropwise with stirring 19.0 g. of 1-benzyl-4-piperidone while maintaining the temperature at -10° to -20°C. Allow the reaction mixture to warm to ambient temperature and stir overnight. Cool the reaction mixture to 10°C. and decompose the reaction mixture with 20% hydrochloric acid (100 ml.). Add ethyl ether to form a two-phase system from which a solid precipitates. Filter, wash the precipitate with saturated sodium chloride and then with ethyl ether and dry *in vacuo*; m.p. 181°-183°C.

Convert the precipitate into the free base with 10% sodium hydroxide. Extract with ethyl ether and treat the ethereal solution with hydrogen chloride in ether to yield the hydrochloride salt as a crystalline precipitate. Recrystallize the product from acetonitrile to obtain thereby the product of this step, m.p. 215°-216°C.

B) 4-Hydroxy-4-(o-methylphenyl)piperidine

Convert 25 g. of the product of step A into the free base with 10% aq. NaOH and ethyl ether. Separate the ethereal solution and concentrate it to an oil weighing 18.3 g. Dissolve the oil in 200 ml. of absolute ethanol and hydrogenate over 4 g. of 20% palladium hydroxide on carbon at 60 psig (4 atmospheres). Remove the catalyst by filtration and concentrate the filtrate to a solid. Recrystallize the product from ethyl acetate to obtain

thereby the product of this step, m.p. 138°-140°C.

C) 4-(o-Methylphenyl-1,2,5,6-tetrahydro)pyridine hydrochloride

Dissolve 2.7 g. of the product of step B in 20 ml. of concentrated hydrochloric acid and 20 ml. of methanol and reflux for three hours. Concentrate the solution to a gummy residue and dry by azeotroping with benzene. Tritrate the solid which which forms during the azeotropic process with ethyl ether, filter, wash with ethyl ether and dry. M.P. 236°-239°C.

D) 2-{3-[1-(1,2,5,6-Tetrahydro)-4-(o-methylphenyl-pyridyl)]-propyl}-1,2,4-triazolo[4,3-a]pyridin-3-(2H)-one

Stir and heat on a steam bath for three hours a mixture of 2.3 g. of the product from step C, 2.5 g. of 2-(3-chloro-propyl)-1,2,4-triazolo [4,3-*a*] pyridine-3-(2H)-one, 0.5 g. of powdered potassium iodide, 5.0 ml. of triethyl-amine and 20 ml. of dimethylformamide. Pour the reaction mixture into water and filter. Dissolve the reddish precipitate in ethyl ether, dry over potassium carbonate, decolorize with activated charcoal and filter. Dilute the ethereal solution (100 ml.) with 50 ml. of hexane and concentrate to 75 ml., cool, and collect the product by filtration; m.p. 106°-107°C.

EXAMPLE 2

2-[3-(4-Phenylpiperidino)-propyl]-1,2,4-triazolo[4,3-a]-pyridin-3-(2H)-one

A) Using the procedure of Coll. Czech. Chem. Comm., 32, 2840 (1967) prepare 4-phenyl-1-(3-chloropropyl)-piperidine.

B) Dissolve 20.4 g. of 1,2,4-triazolo-[4,3-*a*] pyridine-3-(2H)-one in 150 ml. of dimethylformamide, heat the solution to 90°C. and add 7.2 g. of a 50% sodium hydride dispersion in small portions with stirring. Heat and stir for ten minutes and then add 35.7 g. of 4-phenyl-1-(3-chloropropyl)-piperidine in 50 ml. of dimethylformamide dropwise over a 10-15 minute

interval. Stir the reaction mixture on a steam bath for 1 1/2 hours. Cool the reaction mixture and concentrate _in vacuo_. Pour the residue into 500 ml. of water. Filter off the crystalline precipitate, wash it with water and dry it. Recrystallize the product from 500 ml. of a mixture of isopropyl ether, tetrahydrofuran and ethyl ether (about 1:1:1); m.p. $100^o$-$103^oC$. Recrystallize from ethyl ether (1.3 litres); m.p. 103-$5^oC$.; concentrate the mother liquors to obtain further product, m.p. $102^o$-$104^oC$.

## EXAMPLE 3

**2-[3-(4-Phenylpiperidino)-propyl]-1,2,4-triazolo[4,3-a]pyridine-3-(2H)-one**

Dissolve 5.12 g. of 4-phenylpiperidine, 4.5 g. of sodium iodide, 9.0 g. of sodium carbonate (anhydrous) and 7.0 g. of 2-(3-chloropropyl)-1,2,4-triazolo[4,3-_a_] pyridine-3-(2H)-one (prepared as described in U.S. Patent 3,381,009) in 150 ml. of dimethylformamide and heat in an oil bath at $100^o$-$110^oC$. for four hours. Distil off the solvent _in vacuo_. Stir the residue with 100 ml. of water with external cooling. Filter and wash with about 20 ml. of cold water. Dissolve the solid product in 200 ml. of boiling ethyl ether and cool to room temperature. Dry the ethereal solution over anhydrous potassium carbonate, filter and allow the ether to evaporate overnight. Filter off the solid product, wash it with cold ether and dry it.

Dissolve the product in ethyl ether (250 ml.), filter, concentrate to about 100 ml. and chill. Filter off the product to obtain thereby the product of this example, m.p. $102^o$-$103^oC$.

Other compounds of this invention, especially those named above, can be prepared similarly.

The compounds of this invention elicit an analgesic effect

upon administration to warm-blooded animals suffering pain. In conventional tests, such as the mouse writhing test and the rat yeast paw test, the compounds of this invention substantially reduced pain; moreover, they had favourable $ED_{50}$s in the mouse writhing tests. The compounds of this invention advantageously exhibit very low or essentially no hypotensive or tranquilising properties in contrast to many analgesics known in the art.

In most inventions of pharmacologically active compounds, some compounds are more preferred than others. The preference may be based upon economic considerations such as the cost of the starting materials or the ease or difficulty of the synthetic pathways; or may be based upon the degree to which the compounds elicit the desired effect at a given dosage level. Two very preferred compounds of this invention are 2-[3-(4--phenylpiperidino)-propyl]-1,2,4-triazolo[4,3-a]pyridine-3--(2_H_)-one and 2-{(3-[1-(1,2,5,6-tetrahydro-4-_o_-methylphenyl--pyridyl)]-propyl} -1,2,4-triazolo]4,3-a]pyridin-3(2_H_)-one.

Results in the aforementioned tests, particularly the $ED_{50}$ values in the mouse writhing test (which range from about 1.2 mpk to about 5.5 mpk) indicate that the compounds of this invention may be administered about 3 to 4 times a day at a total dosage of from about 50 to about 300 mg./day in divided doses. However, the dose and the frequency of administration will depend upon the patient's general physical condition and age and the cause and degree of the pain and therefore will be determined by the physician.

The compounds of this invention are nitrogen bases and may be administered either as the free base or as an acid addition salt of an inorganic or an organic acid.

For oral administration, the compounds of this invention may be combined with inert pharmaceutical carriers, binders, fillers or excipients such as lactose, mannitol and starch.

For parenteral injection, the compounds may be formulated with an inert, parenterally acceptable vehicle, such as water, saline or sesame oil. The formulations may be compounded according to methods well known to those skilled in the pharmaceutical art. Preferably the compounds are administered in 3-4 daily doses although the specific regimen will be dependent upon the severity and nature of the particular disease state.

The invention therefore provides pharmaceutical compositions comprising, as active ingredient, at least one compound of the formula I defined above or a dehydro derivative thereof having a double bond in the azacyclic ring extending from the carbon atom having the Ar substituent, or a pharmaceutically acceptable acid addition salt, in association with a suitable pharmaceutical carrier or excipient.

These compounds and salts can be administered in the form of dosage units, e.g., injectable dosage units in ampoules but in particular shaped dosage units such as tablets, capsules and suppositories. Dosage units conveniently contain from about 12 to about 300 mg., preferably 25 to 100 mg., of active ingredients. The compounds and salts can also be administered in such oral compositions as elixirs, syrups, solutions and suspensions.

The following data demonstrate relative analgesic activity in rodents for a preferred compound A, of Example 1(D), of this invention and of Trazodone, representing the prior art:

| | mg./kg. $ED_{50}$, orally | |
|---|---|---|
| Test | Compound A | Trazodone |
| Acetic Acid Writhing (mouse) | 1.9 | $>$ 30 |
| Rat Yeast Paw | 4.7 | $>$ 160 |

CLAIMS

1. Compounds of the formula

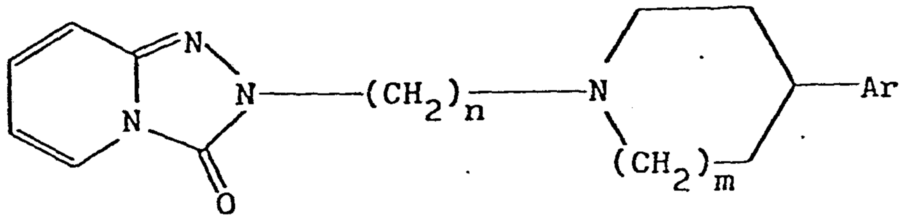

I

wherein Ar is a phenyl group or a phenyl group substituted by one or more members of the group consisting of halogen atoms or lower alkyl, halolower alkyl, hydroxy or lower alkoxy groups, "lower" indicating groups having from 1 to 5 carbon atoms;

$\underline{m}$ is zero or 1, $\underline{n}$ is 2 or 3;

and their dehydro derivatives having a double bond in the azacyclic ring extending from the carbon atom having the Ar substituent;

and the pharmaceutically acceptable acid addition salts thereof.

2. Compounds of the formula:

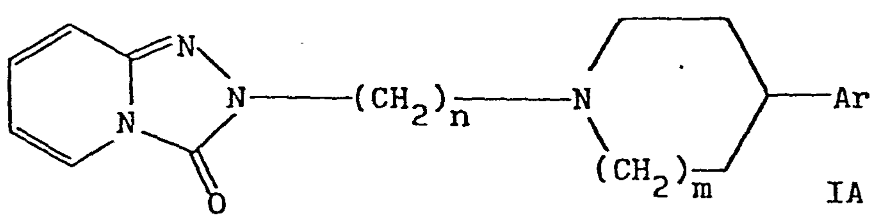

IA

or of the formula:

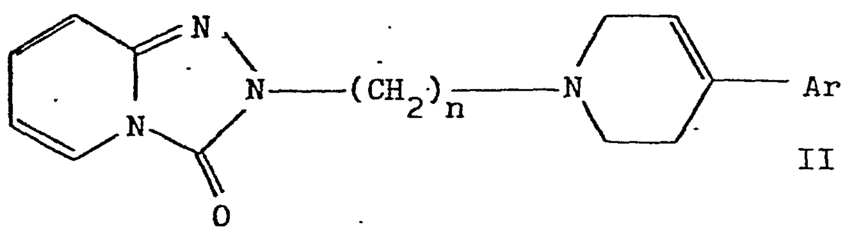

II

wherein Ar, m and n are as defined in claim 1, especially

2-[3-(4-Phenylpiperidino)-propyl]-1,2,4-triazolo[4,3-a] pyridin-3-(2H)-one and 2-{3-[1-(1,2,5,6-tetrahydro-4-o-methylphenyl-pyridyl)]-propyl}--1,2,4-triazolo[4,3-a]pyridin-3-(2H)-one.

3. 2-{3-[1-(1,2,5,6-Tetrahydro-4-p-chlorophenyl-pyridyl)]--propyl}-1,2,4-triazolo[4,3-a]pyridin-3-(2H)-one,
2-{3-[1-(1,2,5,6-tetrahydro-4-p-methylphenyl-pyridyl)]-propyl}-1,2,4-triazolo[4,3-a]pyridin-3-(2H)-one,
2-[3-(4-p-chlorophenyl-piperidino)-propyl]-1,2,4-triazolo[4,3--a]pyridin-3-(2H)-one,
2-[3-(4-m-chlorophenyl-piperidino)-propyl]-1,2,4-triazolo-[4,3-a]pyridin-3-(2H)-one,
2-[3-(4-p-methylphenyl-piperidino)-propyl]-1,2,4-triazolo[4,3-a] pyridin-3-(2H)-one,
2-[3-(4-m-methylphenyl-piperidino)-propyl]-1,2,4-triazolo[4,3--a]pyridin-3-(2H)-one,
2-{3-[1-(1,2,5,6-tetrahydro-4-m-chlorophenylpyridyl)]-propyl}--1,2,4-triazolo[4,3-a]pyridin-3-(2H)-one,
2-[3-(4-p-fluorophenyl-piperidino)-propyl]-1,2,4-triazolo[4,3-

-a]pyridin-3-[2H)-one, 2-[3-(4-p-methoxyphenyl-piperidino)-propyl]-1,2,4-triazolo [4,3-a]pyridin-3-(2H)-one, 2-{3-[1-(3-phenylpyrrolidinyl)]-propyl}-1,2,4-triazolo[4,3-a] pyridin-3-(2H)-one, 2-{3-[1-(1,2,5,6-tetrahydro-3-o-trifluoromethylphenyl-pyridyl)]-propyl}-1,2,4-triazolo[4,3-a]pyridin-3-(2H)-one, and 2-[3-(4-m-hydroxyphenyl-piperidino)-propyl]-1,2,4-triazolo[4,3-a] pyridin-3-(2H)-one.

4. Compounds as claimed in any of claims 1 to 3 in the form of their acid addition salts with hydrochloric, hydrobromic, nitric, sulfuric, maleic, fumaric, tartaric, citric or succinic acid.

5. A process for the preparation of a compound or salt as claimed in any of claims 1 to 4 which comprises either a) reacting a compound of the formula

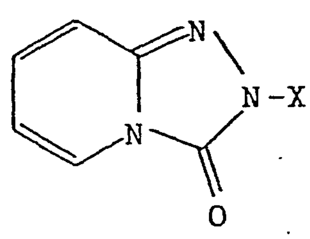

III

with a compound of the formula

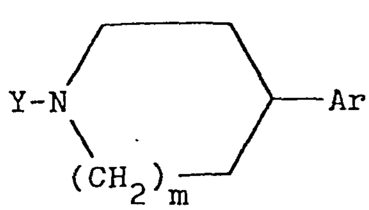

IV

or with a dehydro derivative thereof having a double bond in the azacyclic ring extending from the carbon atom having the Ar substituent;

wherein one of X and Y is a group of the formula

$$-(CH_2)_n-Z$$

where Z is a reactive ester group,
and the other of X and Y is an atom or group capable of being eliminated with Z;
and m, n and Ar are as defined above;
or

b) -- for the preparation of a dehydro derivative of a compound of the formula I having a double bond in the azacyclic ring extending from the carbon atom having the Ar substituent -- eliminating a compound of the formula WH from a compound of the formula

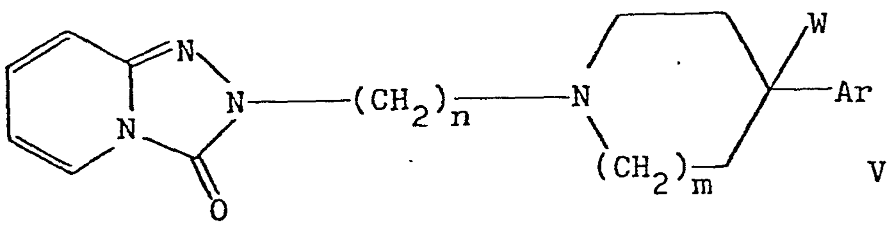

V

wherein m, n and Ar are as defined in claim 1 and W is a hydroxy group or an esterified hydroxy group;

whereafter the product of the formula I is isolated as the free base or as a pharmaceutically acceptable acid addition salt thereof.

6. A process as claimed in claim 5, process a), wherein X is a hydrogen atom and Y is the group $-(CH_2)_n-Z$, where Z is a halogen atom, especially chlorine, and n is as defined above.

7. A process as claimed in claim 5, process a), wherein X is the group of the formula $-(CH_2)_n-Z$, where Z is a halogen atom, especially chlorine, and n is as defined above, and Y is a hydrogen atom or a group that can be displaced in the reaction, e.g. benzyl.

8. A process as claimed in claim 5, process b), wherein W is a hydroxy group and the elimination of the compound of the formula WH is effected at moderate temperature under mildly acidic conditions.

9. Pharmaceutical compositions containing as active ingredient at least one compound or salt as claimed in any of claims 1 to 4 together with a pharmaceutical carrier or excipient, especially such compositions in the form of dosage units such as tablets, capsules or suppositories, which preferably contain from 12 to 300 mg., in particular 25 to 100 mg., of active ingredient per dosage unit.

10. Compositions as claimed in claim 9 in the form of injectable compositions.

11. Compositions as claimed in claim 9 or claim 10 wherein the active ingredient is a compound named in claim 2.

European Patent Office

0062873

Application number

# EUROPEAN SEARCH REPORT

EP 82 10 2893

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | GB-A-1 117 068 (A.FRANCESCO) *Claim 1; page 1, lines 17-31, page 2, lines 1-31*<br>----- | 1,9 | C 07 D 471/04<br>A 61 K 31/44 //<br>C 07 D 211/52<br>C 07 D 211/70<br>(C 07 D 471/04<br>C 07 D 249/00<br>C 07 D 221/00 ) |
| | | | TECHNICAL FIELDS SEARCHED (Int. Cl. ³) |
| | | | C 07 D 471/00<br>A 61 K 31/00 |

The present search report has been drawn up for all claims

| Place of search | Date of completion of the search | Examiner |
|---|---|---|
| THE HAGUE | 15-06-1982 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82